# FASCICULE DE BREVET EUROPEEN

(11) **EP 2 861 206 B1**
(45) Date de publication et mention de la délivrance du brevet: **12.06.2019**
(21) Numéro de dépôt: 13731415.9
(22) Date de dépôt: 12.06.2013
(51) Int. Cl.: A61K 8/49, A61Q 5/00, A61Q 7/00, A61K 8/81, A61Q 5/02, A61Q 5/06, A61Q 5/12, A61K 8/84

(54) **COMPOSITION COMPRENANT UN ESTER D'ACIDE PYRIDINE DICARBOXYLIQUE ET UN POLYMERE FIXANT, PROCEDE ET UTILISATION POUR TRAITER LES CHEVEUX**
ZUSAMMENSETZUNG MIT EINEM PYRIDINDICARBONSÄUREESTER UND EINEM BINDUNGSPOLYMER, VERFAHREN DAFÜR UND VERWENDUNG ZUR BEHANDLUNG VON HAAREN
COMPOSITION INCLUDING A PYRIDINE DICARBOXYLIC ACID ESTER AND A BINDING POLYMER, METHOD THEREFOR, AND USE THEREOF FOR TREATING HAIR

(30) Priorité: 15.06.2012 FR 1255613; 31.08.2012 US 201261695336 P
(43) Date de publication de la demande: 22.04.2015
(73) Titulaire: L'OREAL, 75008 Paris (FR)
(72) Inventeur: DRILLON, Damien, 93400 SAINT-OUEN (FR); DERKX, Tiphaine, F-75017 Paris (FR); RICHET, Laurence, F-95590 Presles (FR)
(74) Mandataire: Dodin, Catherine
(86) Numéro de dépôt international: PCT/FR2013/051364
(87) Numéro de publication internationale: WO 2013/186486

(56) Documents cités:
- EP-A1- 1 352 629
- EP-A2- 0 489 581
- WO-A1-03/066010
- WO-A1-2007/061169
- DE-A1-102006 050 398

## Description

La présente invention concerne une composition cosmétique ou pharmaceutique comprenant au moins un dérivé d'acide pyridine-dicarboxylique en association avec au moins un polymère fixant, ainsi que son utilisation notamment dans le domaine capillaire.

La croissance des cheveux et leur renouvellement sont principalement déterminés par l'activité des follicules pileux et leur environnement matriciel. Leur activité est cyclique et comporte essentiellement trois phases à savoir la phase anagène, la phase catagène et la phase télogène. La chevelure se renouvelle en permanence et sur les 150 000 cheveux environ que comporte une chevelure, 10% environ sont au repos et seront remplacés dans les mois qui viennent.
La chute ou perte naturelle des cheveux peut être estimée, en moyenne, à quelques cent cheveux par jour pour un état physiologique normal. Ce processus de renouvellement physique permanent subit une évolution naturelle au cours du vieillissement, les cheveux deviennent plus fins et leurs cycles plus courts.
Il est connu, par ailleurs, que certains facteurs tels qu'un déséquilibre hormonal, un stress physiologique, la malnutrition, peuvent accentuer le phénomène. En outre, la chute et/ou l'altération des cheveux peuvent être en relation avec des phénomènes saisonniers.
Il a ainsi notamment été proposé par le brevet EP1352629, d'utiliser des dérivés d'acide pyridine-dicarboxylique pour induire et/ou stimuler la croissance des fibres kératiniques humaines telles que les cheveux ou les cils, et/ou freiner leur chute et/ou augmenter leur densité.

Toutefois, on a constaté que l'utilisation de tels composés, particulièrement en présence de solvant, pouvait entraîner des sensations d'inconfort du cuir chevelu, telles que des tiraillements, ou encore des problèmes de sécheresse. Dans certains cas, on a également observé des cheveux ternes, sans tonus, ou difficiles à coiffer et à discipliner.

La présente invention a notamment pour but de pallier ces inconvénients en proposant une composition permettant d'obtenir un effet bénéfique le plus étendu possible, ladite composition agissant de manière large sur le cuir chevelu et l'ensemble de la chevelure, du bulbe du cheveu à sa pointe.
Grâce à l'invention, on peut ainsi obtenir d'une part l'effet technique lié à l'utilisation des dérivés d'acide pyridine-dicarboxylique, tout en ayant un bon effet cosmétique sur l'ensemble de la chevelure.
On a constaté que l'association selon l'invention permet d'obtenir, d'une part, une répartition et un étalement de la composition sur la chevelure améliorés, et d'autre part, une amélioration des propriétés de conditionnement des cheveux, notamment en terme de douceur, souplesse, lissage et démêlage.

La présente invention a pour objet une composition comprenant :
- (i) au moins un composé de formule générale (I), ou l'un de ses sels : 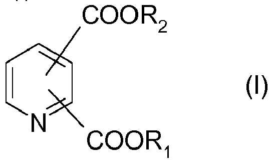 dans laquelle R₁ et R₂ représentent, indépendamment l'un de l'autre, un atome d'hydrogène, un radical hydrocarboné aliphatique en C₁-C₁₈, linéaire ou ramifié, saturé ou insaturé (alkyle notamment); ou un radical aryle en C6-C18;
   lesdits radicaux hydrocarboné aliphatique ou aryle étant éventuellement substitués par un ou plusieurs groupes choisis parmi OH, NH₂, -OR, -OCOR et -NHR avec R représentant un groupement alkyle en C1-C18,
   l'un au moins des groupements R1 et R2 étant différent d'un atome d'hydrogène; et
- (ii) au moins un polymère fixant choisi parmi les copolymères vinylpyrrolidone/acrylate ou méthacrylate de dialkylaminoalkyle quaternisés ou non, les copolymères d'acides ou d'anhydrides carboxyliques monoinsaturés en C4-C8, les homopolymères acrylamido-alkylsulfonique.

On a constaté que les compositions selon l'invention présentaient de bonnes performances cosmétiques, au niveau de la qualité des cheveux, et également au niveau du confort du cuir chevelu. Le cuir chevelu est apaisé, et aucun tiraillement ou échauffement n'est observé après l'application de la composition. La chevelure est disciplinée, facile à coiffer, les cheveux sont plus forts et plus résistants.

Dans la présente description, l'expression "au moins un" est équivalente à l'expression "un ou plusieurs" et peut y être substituée; et l'expression "compris entre ... et ..." est équivalente à l'expression "allant de ... à ..." et peut y être substituée.

### 1/ Ester d'acide pyridine dicarboxylique

La composition selon l'invention comprend donc au moins un composé qui est un ester d'acide pyridine dicarboxylique. L'ester peut être un monoester ou un diester, de préférence un diester. Il peut également s'agir d'un sel d'un tel ester.
Lesdits composés répondent à la formule générale (I), ou l'un de ses sels : 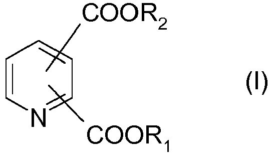 dans laquelle R₁ et R₂ représentent, indépendamment l'un de l'autre, un atome d'hydrogène, un radical hydrocarboné aliphatique en C₁-C₁₈, linéaire ou ramifié, saturé ou insaturé (alkyle notamment); ou un radical aryle en C6-C18;
lesdits radicaux hydrocarboné aliphatique ou aryle étant éventuellement substitués par un ou plusieurs groupes choisis parmi OH, NH₂, -OR, -OCOR et -NHR avec R représentant un groupement alkyle en C1-C18,
l'un au moins des groupements R1 et R2 étant différent d'un atome d'hydrogène.

De préférence, les substituants COOR₁ et COOR₂ sont respectivement en position 2 et 3, ou 2 et 4 du noyau pyridine. Préférentiellement, ils sont en position 2 et 4.

Préférentiellement, les composés répondent à la formule (la), ou un de ses sels : 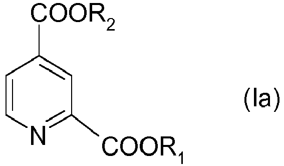 dans laquelle R₁ et R₂ représentent, indépendamment l'un de l'autre, un atome d'hydrogène, un radical hydrocarboné aliphatique en C₁-C₁₈, linéaire ou ramifié, saturé ou insaturé (alkyle notamment); ou un radical aryle en C6-C18;
lesdits radicaux hydrocarboné aliphatique ou aryle étant éventuellement substitués par un ou plusieurs groupes choisis parmi OH, NH₂, -OR, -OCOR et -NHR avec R représentant un groupement alkyle en C1-C18,
l'un au moins des groupements R1 et R2 étant différent d'un atome d'hydrogène.

De préférence, dans les formules (I) et (la), le radical hydrocarboné aliphatique, notamment alkyle, en C₁-C₁₈ est un radical hydrocarboné aliphatique, notamment alkyle, en C1-C10, notamment en C1-C6, tel que méthyle, éthyle, tertiobutyle, iso-propyle, hexyle. Ledit radical hydrocarboné aliphatique peut également contenir au moins une double liaison ou une triple liaison carbone-carbone, comme par exemple -CH=CH₂, -CH₂-CH=CH-CH₃ et -CH₂-C=CH.
Le radical aryle peut représenter le radical phényle ou naphtyle.

En particulier, dans les formules (I) et (la), R1 et R2 représentent, indépendamment l'un de l'autre, un atome d'hydrogène, un radical hydrocarboné aliphatique saturé, notamment alkyle, linéaire ou ramifié, en C₁-C₁₈, mieux en C1-C10, voire en C1-C6, éventuellement substitué par un groupe alcoxy ou acyloxy (OR ou OCOR avec R désignant un radical alkyle en C1-C18), l'un au moins des groupements R1 et R2 étant différent d'un atome d'hydrogène.

De préférence, R1 et R2 sont identiques.
Préférentiellement, R₁ et R₂ sont identiques et représentent un radical alkyl saturé et linéaire en C1-C18, de préférence en C1-C10, et encore mieux en C1-C6; et tout particulièrement un radical éthyle.

On peut notamment utiliser les composés de formule (I) suivants :
- le pyridine-2,4-dicarboxylate de diméthyle,
- le pyridine-2,3-dicarboxylate de diméthyle,
- le pyridine-2,4-dicarboxylate de diéthyle,
- le pyridine-2,3-dicarboxylate de diéthyle,
- le pyridine-2,5-dicarboxylate de diéthyle,
- le pyridine-2,5-dicarboxylate de diméthyle,
- le pyridine-2,4-dicarboxylate de diisopropyle,
- le pyridine-2,4-dicarboxylate de di(acétyloxyméthyle) (dérivé de formule (I) tel que R₁ et R₂ représentent -CH₂-O-COCH₃),
Préférentiellement, la composition comprend, comme composé de formule (I) ou (la), le pyridine-2,4-dicarboxylate de diéthyle.

Par sels des composés de formule (I), on entend selon l'invention les sels organiques ou minéraux d'un composé de formule (I), ces sels étant physiologiquement acceptables. Comme sels minéraux, on peut citer les sels de sodium ou de potassium ainsi que les sels de zinc (Zn²⁺), de calcium (Ca²⁺), de cuivre (Cu²⁺), de fer (Fe²⁺), de strontium (Sr²⁺), de magnésium (Mg²⁺), de manganèse (Mn²⁺); les hydroxydes, les carbonates et les chlorures. Comme sels organiques, on peut citer les sels triéthanolamine, mono-éthanolamine, diéthanolamine, hexadécylamine, N,N,N',N'-tétrakis-(hydroxy-propyl-2) éthylène di-amine et tris-hydroxyméthyl aminométhane.

Les composés de formule (I) sont connus en tant que tels; ils sont notamment décrits, ainsi que leur fabrication, dans le brevet EP1352629.

De préférence, la composition selon l'invention comprend le ou les composés de formule (I) en une quantité comprise entre 0,001 et 10% en poids, notamment de 0,01 à 5% poids, par rapport au poids total de la composition.

### 2/ Polymères fixants

La composition selon l'invention comprend également au moins un polymère fixant choisi parmi les copolymères vinylpyrrolidone/acrylate ou méthacrylate de dialkylaminoalkyle quaternisés ou non, les copolymères d'acides ou d'anhydrides carboxyliques monoinsaturés en C4-C8, les homopolymères acrylamido-alkylsulfonique..

Au sens de l'invention, on entend par polymère fixant tout polymère susceptible, par application sur les cheveux, de conférer une forme à la chevelure ou de permettre le maintien d'une forme déjà acquise.

De préférence, le ou les polymères fixants sont hydrodispersibles ou hydrosolubles; préférentiellement, le polymère fixant est hydrosoluble.

Par polymère hydrosoluble, on entend au sens de la présente invention un polymère qui, à pH 7 et à 25°C, 1 atm., présente une solubilité pondérale dans l'eau supérieure ou égale à 0,1%, mieux supérieure ou égale à 0,5%, encore mieux supérieure ou égale à 1%.

De préférence, la composition selon l'invention comprend le ou les polymères fixants en une quantité comprise entre 0,01 et 10% en poids, notamment 0,01 et 5% en poids, encore mieux entre 0,015 et 1% en poids, par rapport au poids total de la composition.

### 3. Ingrédients complémentaires

La composition selon l'invention peut être à usage cosmétique ou pharmaceutique. Préférentiellement, la composition selon l'invention est à usage cosmétique. Par "cosmétique", on entend au sens de l'invention une composition d'aspect, d'odeur et de toucher agréables. De préférence, la composition est d'application topique sur la peau et les fibres kératiniques, et plus spécialement sur le cuir chevelu, les cheveux et les cils.

Selon le mode d'application, cette composition peut se présenter sous toutes les formes galéniques normalement utilisées dans le domaine considéré, notamment cosmétique.

Pour une application topique sur la peau, y compris le cuir chevelu, la composition peut avoir la forme notamment d'une solution ou suspension aqueuse, alcoolique ou hydroalcoolique, d'une suspension ou d'une solution huileuse, d'une émulsion ou dispersion de consistance liquide ou semi-liquide obtenue par dispersion d'une phase grasse dans une phase aqueuse (H/E) ou inversement (E/H), d'une dispersion ou émulsion de consistance molle, d'un gel aqueux ou hydroalcoolique ou huileux (anhydre), d'une poudre libre ou compactée à utiliser telle quelle ou à incorporer dans un milieu physiologiquement acceptable (excipient), ou encore de microcapsules ou microparticules, ou de dispersions vésiculaires de type ionique et/ou non ionique.
On peut également envisager une composition sous la forme d'une mousse ou encore sous forme de composition pour aérosol comprenant également un agent propulseur sous pression.

En particulier, la composition selon l'invention peut être une composition capillaire, susceptible d'être appliquée sur le cuir chevelu ou les cheveux, et peut se présenter sous forme d'une lotion de soin capillaire, par exemple d'application journalière ou bihebdomadaire, d'un shampooing ou d'un après-shampooing capillaire, en particulier d'application bihebdomadaire ou hebdomadaire, d'un savon liquide ou solide de nettoyage du cuir chevelu d'application journalière, d'un produit de mise en forme de la coiffure (laque, produit pour mise en pli, gel coiffant), d'un masque traitant, d'une crème ou d'un gel moussant de nettoyage des cheveux. Elle peut encore se présenter sous forme de teinture ou de mascara capillaire à appliquer au pinceau ou au peigne.
Pour une application sur les cils ou les poils, la composition selon l'invention peut se présenter sous forme d'un mascara, pigmenté ou non, à appliquer à la brosse sur les cils ou encore sur les poils de barbe ou de moustache.
Selon un mode de réalisation particulier, la composition selon l'invention se présente sous forme de crème ou lotion capillaire, de shampooing, d'après-shampooing capillaire, ou de mascara capillaire ou pour cils.

Les quantités des différents constituants de la composition selon l'invention sont celles classiquement utilisées dans les domaines considérés. En outre, cette composition est préparée selon les méthodes usuelles. Elle peut ainsi se présenter sous forme d'une lotion, sérum, lait, crème H/E ou E/H, gel, onguent, pommade, poudre, baume, patch, tampon imbibé, savon, pain, mousse.

Lorsque la composition est une émulsion, la proportion de la phase grasse peut aller de 2 à 80% en poids, de préférence de 5 à 50% en poids par rapport au poids total de la composition.
La phase grasse peut contenir des composés gras ou huileux, liquides à température ambiante (25°C) et pression atmosphérique (1 atm), généralement appelés huiles. Ces huiles peuvent être compatibles ou non entre elles et former une phase grasse liquide macroscopiquement homogène ou un système bi- ou triphasique.
La phase grasse peut, en plus des huiles, contenir des cires, des gommes, des polymères lipophiles, des produits "pâteux" ou visqueux contenant des parties solides et des parties liquides.

La phase aqueuse est ajustée en fonction de la teneur en phase grasse et en composé(s) de formule (I) ainsi que de celle des éventuels ingrédients additionnels, pour obtenir 100% en poids. En pratique la phase aqueuse représente de préférence 5 à 99,9% en poids.
La phase aqueuse contient de l'eau et éventuellement un solvant organique miscible en toute proportion à l'eau comme les alcools inférieurs en C₁ à C₈ tel que l'éthanol, l'isopropanol, les polyols comme le propylène glycol, le glycérol, le sorbitol ou encore l'acétone.

Les émulsionnants et les coémulsionnants utilisés dans la composition sous forme d'émulsion peuvent être choisis parmi ceux classiquement utilisés dans le domaine cosmétique ou pharmaceutique. L'émulsionnant et/ou le coémulsionnant sont de préférence présents dans la composition en une proportion allant de 0,1 à 30% en poids, de préférence de 0,5 à 20% en poids par rapport au poids total de la composition, mieux de 1 à 8%. Leur nature est, en outre, fonction du sens de l'émulsion. L'émulsion peut, en outre, comprendre des vésicules lipidiques et notamment des liposomes.

Lorsque la composition est une solution ou un gel huileux, la phase grasse peut représenter plus de 90% du poids total de la composition.

Avantageusement, pour une application capillaire, la composition est une solution ou suspension aqueuse, alcoolique ou hydroalcoolique, et mieux une solution ou une suspension eau/éthanol. La fraction alcoolique peut représenter de 5% à 99,9% et mieux de 8% à 80%.
Pour une application mascara, la composition est de préférence une dispersion de cire-dans-eau ou de cire-dans-huile, une huile gélifiée ou un gel aqueux, pigmenté ou non.

Dans un mode de réalisation particulier, la composition selon l'invention peut comprendre au moins une vitamine B et/ou un de ses analogues ou dérivés.
Par vitamine B, on entend au moins l'une des vitamines appartenant à ce groupe, à savoir la vitamine B1 ou thiamine; la vitamine B2 ou riboflavine; la vitamine B3 (ou vitamine PP) ou niacine ou niacinamide; la vitamine B5 ou acide pantothénique; la vitamine B6 ou pyridoxine; la vitamine B8 (ou vitamine H) ou biotine; la vitamine B9 ou acide folique, et la vitamine B12 ou cyanocobalamine.

Comme analogues ou dérivés de vitamine B, on peut notamment citer les sels correspondants, comme le pantothénate de calcium, les pro-vitamines B comme le panthénol, qui est l'alcool analogue de la vitamine B5, encore appelé provitamine B5; on peut également citer les éthers et les esters correspondants, comme le panthényl éthyl éther, le panthényl éthyl éther acétate et le panthényl triacétate. On peut bien évidemment utiliser un mélange de ces différents composés. Préférentiellement, la vitamine B susceptible d'être employée dans le cadre de l'invention est choisie parmi la vitamine B3, la vitamine B5, la vitamine B6 et la vitamine B8, ou l'un de leurs analogues ou dérivés, et notamment le pantothénate de calcium, le panthénol, le panthényl éthyl éther, le panthényl éthyl éther acétate et le panthényl triacétate; ainsi que leurs mélanges.
De préférence, la composition selon l'invention comprend la ou les vitamines B, analogue(s) et/ou dérivé(s) en une quantité comprise entre 0,001 et 5% en poids, notamment 0,005 et 2% en poids, encore mieux entre 0,01 et 1% en poids, par rapport au poids total de la composition.

La composition selon l'invention peut également comprendre des adjuvants habituels dans le domaine cosmétique, choisis par exemple parmi les gélifiants ou épaississants hydrophiles ou lipophiles, les additifs hydrophiles ou lipophiles, les conservateurs, les antioxydants (caroténoïdes), les parfums, les charges, les absorbeurs d'odeurs, les électrolytes, les neutralisants, les agents bloqueurs d'U.V. comme les filtres solaires, les polymères filmogènes différents des polymères fixants, les actifs cosmétiques comme les vitamines; les matières colorantes, solubles ou non dans le milieu. Les quantités de ces différents adjuvants sont celles classiquement utilisées dans le domaine cosmétique, par exemple de 0,01 à 20%, mieux de 0,1 à 10% du poids total de la composition. Ces adjuvants, selon leur nature, peuvent être introduits dans la phase grasse, dans la phase aqueuse et/ou dans les vésicules lipidiques et notamment des liposomes.

Comme huiles ou cires susceptibles d'être employées, on peut citer les huiles minérales (huile de vaseline, isoparaffine hydrogénée), les huiles végétales (fraction liquide du beurre de karité, huile de tournesol, de soja, de germes de blé), les huiles animales (perhydrosqualène), les huiles de synthèse (huile de Purcellin, esters d'acide gras), les huiles ou cires siliconées (polydiméthylsiloxanes linéaires ou cycliques, cyclométhicone, phényltriméthicone), les huiles fluorées (perfluoropolyéthers), les cires d'abeille, de Candellila, de riz, de carnauba, de paraffine ou de polyéthylène. On peut également citer des alcools gras et des acides gras (acide stéarique, linoléique, linolénique par exemple).

Comme émulsionnants utilisables dans l'invention, on peut citer par exemple les alcools gras oxyéthylénés, le stéarate ou laurate de glycérol, le stéarate ou oléate de sorbitol polyoxyéthyléné (par exemple le polysorbate 60 et le mélange de PEG-6/PEG-32/Glycol Stéarate vendu sous la dénomination de Tefose® 63 par la société Gattefosse), les (alkyl)diméthicones copolyol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les Bentones, les sels métalliques d'acides gras comme les stéarates d'aluminium, la silice traitée hydrophobe, l'éthylcellulose et leurs mélanges.

La composition peut comprendre d'autres actifs qui peuvent être hydrophiles, comme les protéines ou les hydrolysats de protéine, les acides aminés, les polyols, l'urée, l'allantoïne, les sucres et les dérivés de sucre, les vitamines hydrosolubles, les extraits végétaux, ces extraits pouvant alors contenir ou non des isoflavones; ou qui peuvent être lipophiles, comme le rétinol (vitamine A) et ses dérivés notamment ester (palmitate), le tocophérol (vitamine E) et ses dérivés, notamment ester (acétate palmitate), les acides gras essentiels comme les acides eicosatétraénoïque et eicosatriénoïque ou leurs esters et amides, les céramides, les huiles essentielles, les esters des hydroxyacides, les phospholipides comme la lécithine; ou qui peuvent être solubles dans des solvants alcooliques comme les lactones (kawaïne); et leurs mélanges.
Elle peut également comprendre des actifs additionnels notamment ceux favorisant la repousse des fibres kératiniques humaines et/ou limitant leur chute. On peut notamment citer, seul ou en mélange :
- les agents antibactériens; les antiparasitaires, les antifongiques;
- les extraits de micro-organismes notamment les extraits bactériens ;
- les agents modulant la différenciation et/ou la prolifération cutanée tels que l'acide rétinoïque et ses isomères, le rétinol et ses esters, la vitamine D et ses dérivés, les oestrogènes tels que l'oestradiol ;
- les agents modulant l'adhésion bactérienne sur la peau et /ou les muqueuses tels que le miel, notamment le miel d'acacias et certains dérivés de sucres ;
- l'acide acétylsalicylique, l'acétaminophène ou l'acide glycyrrhétinique, l'a-bisabolol;
- les agents kératolytiques tels que les acides alpha- et bêta-hydroxycarboxyliques ou bêta-cétocarboxyliques, leurs sels, amides ou esters, les lactones et leurs sels correspondants, et plus particulièrement les hydroxyacides tels que l'acide glycolique, l'acide lactique, l'acide salicylique, l'acide citrique et de manière générale les acides de fruits, les dérivés de l'acide salicylique tels que ceux porteurs d'un radical alcanoyle ayant de 2 à 12 atomes de carbone en position 5 du cycle benzénique comme l'acide n-octanoyl-5-salicylique ;
- les agents anti-radicaux libres tels que l'alpha-tocophérol ou ses esters, les superoxyde dismutases, certains chélatants de métaux ou l'acide ascorbique et ses esters ;
- les anti-séborrhéiques;
- les antipelliculaires comme l'octopirox ou la pyrithione de zinc ;
- les agents diminuant la chute des cheveux comme l'aminexil, le 6-0-[(9Z,12Z)-octadéca-9,12-diènoyl]hexapyranose; les dérivés de pyrimidine, comme le 2,4-diamino 6-pipéridinopyrimidine 3-oxyde ou "minoxidil" décrit dans les brevets US4139619 et US4596812.

La composition selon l'invention peut en outre comprendre des substances telles que les antagonistes de substance P, de CGRP ou de bradykinine et les inhibiteurs de NO-synthase, composés décrits comme étant actifs dans le traitement des peaux sensibles et comme présentant des effets anti-irritants, en particulier vis-à-vis de composés irritants éventuellement présents dans les compositions.

La composition selon l'invention, en particulier cosmétique, peut être appliquée sur les zones alopéciques du cuir chevelu et des cheveux, éventuellement laissée en contact plusieurs heures et/ou éventuellement rincée.
On peut, par exemple, appliquer la composition selon l'invention le soir, garder celle-ci en contact toute la nuit et éventuellement effectuer un shampooing le matin. Ces applications peuvent être renouvelées quotidiennement pendant un ou plusieurs mois suivant les individus.

La présente invention a également pour objet un procédé de traitement cosmétique des matières kératiniques humaines, notamment des cheveux et/ou de la peau, y compris le cuir chevelu, dans lequel on applique sur lesdites matières kératiniques une composition cosmétique telle que définie ci-dessus, et éventuellement à rincer lesdites matières kératiniques.
Plus spécialement, ledit procédé est un procédé de soin cosmétique des cheveux et/ou du cuir chevelu, en vue d'améliorer leur état et/ou leur aspect.
Ce procédé de traitement présente bien les caractéristiques d'un procédé cosmétique dans la mesure où il permet d'améliorer l'esthétique des matières, notamment des fibres, kératiniques humaines en leur donnant une plus grande vigueur, un aspect amélioré (apport de conditionnement), une plus grande facilité de manipulation (étalement, répartition) et de coiffage.

L'invention est décrite plus en détail dans les exemples suivants.

### Exemple 1

On prépare la composition suivante (% en poids, MA = matière active):

| | |
|---|---|
| - diéthyl ester d'acide pyridine-2,4-dicarboxylique (nom INCI : DIETHYLLUTIDINATE) | 5% |
| - Polyquaternium 11 (copolymère vinylpyrrolidone/ méthacrylate de diméthylaminoéthyle quaternisé) | 0,02% MA |
| - éthanol | 56,5% |
| - acide citrique | qs pH = 5,5 |
| - eau | qsp 100% |

Cette lotion est limpide et stable dans le temps (au moins 2 mois, à 25°C ou 45°C).

Appliquée raie par raie sur le cuir chevelu, cette lotion présente de bonnes qualités d'usage en favorisant la distribution et la répartition des actifs sur le cuir chevelu. Elle présente également de bonnes performances cosmétiques. Les cheveux sont soyeux et légers, la chevelure est disciplinée et facile à coiffer.

## Revendications

1. Composition comprenant :
- (i) au moins un composé de formule générale (I), ou l'un de ses sels : 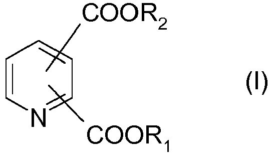 dans laquelle R₁ et R₂ représentent, indépendamment l'un de l'autre, un atome d'hydrogène, un radical hydrocarboné aliphatique en C₁-C₁₈, linéaire ou ramifié, saturé ou insaturé (alkyle notamment); ou un radical aryle en C6-C18;
lesdits radicaux hydrocarboné aliphatique ou aryle étant éventuellement substitués par un ou plusieurs groupes choisis parmi OH, NH₂, -OR, -OCOR et -NHR avec R représentant un groupement alkyle en C₁-C₁₈,
l'un au moins des groupements R1 et R2 étant différent d'un atome d'hydrogène; et
- (ii) au moins un polymère fixant choisi parmi les copolymères vinylpyrrolidone/acrylate ou méthacrylate de dialkylaminoalkyle quaternisés ou non, les copolymères d'acides ou d'anhydrides carboxyliques monoinsaturés en C4-C8, les homopolymères acrylamido-alkylsulfonique.

2. Composition selon la revendication 1, dans laquelle les substituants COOR₁ et COOR₂ sont respectivement en position 2 et 3, ou 2 et 4 du noyau pyridine; préférentiellement en position 2 et 4.

3. Composition selon l'une des revendications précédentes, dans laquelle R1 et R2 sont identiques.

4. Composition selon l'une des revendications précédentes, dans laquelle R1 et R2 représentent, indépendamment l'un de l'autre, un atome d'hydrogène, un radical hydrocarboné aliphatique saturé, notamment alkyle, linéaire ou ramifié, en C₁-C₁₈, mieux en C1-C10, voire en C1-C6, éventuellement substitué par un groupe alcoxy ou acyloxy (OR ou OCOR avec R désignant un radical alkyle en C1-C18), l'un au moins des groupements R1 et R2 étant différent d'un atome d'hydrogène.

5. Composition selon l'une des revendications précédentes, dans laquelle R₁ et R₂ sont identiques et représentent un radical alkyl saturé et linéaire en C1-C18, de préférence en C1-C10, et encore mieux en C1-C6; et tout particulièrement un radical éthyle.

6. Composition selon l'une des revendications précédentes, comprenant comme composé de formule (I), l'un des composés suivants :
- le pyridine-2,4-dicarboxylate de diméthyle,
- le pyridine-2,3-dicarboxylate de diméthyle,
- le pyridine-2,4-dicarboxylate de diéthyle,
- le pyridine-2,3-dicarboxylate de diéthyle,
- le pyridine-2,5-dicarboxylate de diéthyle,
- le pyridine-2,5-dicarboxylate de diméthyle,
- le pyridine-2,4-dicarboxylate de diisopropyle,
- le pyridine-2,4-dicarboxylate de di(acétyloxyméthyle) (dérivé de formule (I) tel que R₁ et R₂ représentent -CH₂-O-COCH₃),

7. Composition selon l'une des revendications précédentes, dans laquelle le ou les composés de formule (I) sont présents en une quantité comprise entre 0,001 et 10% en poids, notamment de 0,01 à 5% poids, par rapport au poids total de la composition.

8. Composition selon l'une des revendications précédentes, dans laquelle le ou les polymères fixants sont présents en une quantité comprise entre 0,01 et 10% en poids, notamment 0,01 et 5% en poids, encore mieux entre 0,015 et 1% en poids, par rapport au poids total de la composition.

9. Composition selon l'une des revendications précédentes, comprenant en outre au moins une vitamine B et/ou un de ses analogues ou dérivés; et notamment choisi parmi la vitamine B3, la vitamine B5, la vitamine B6 et la vitamine B8, ou l'un de leurs analogues ou dérivés, et plus particulièrement parmi le pantothénate de calcium, le panthénol, le panthényl éthyl éther, le panthényl éthyl éther acétate et le panthényl triacétate, ainsi que leurs mélanges.

10. Composition selon la revendication 9, comprenant la ou les vitamines B, analogue(s) et/ou dérivé(s) en une quantité comprise entre 0,001 et 5% en poids, notamment 0,005 et 2% en poids, encore mieux entre 0,01 et 1% en poids, par rapport au poids total de la composition.

11. Composition selon l'une des revendications précédentes, se présentant sous forme d'une composition cosmétique capillaire, telle qu'une lotion de soin capillaire, d'un shampooing ou d'un après-shampooing capillaire, d'un savon liquide ou solide de nettoyage du cuir chevelu, d'un produit de mise en forme de la coiffure, d'un masque traitant, d'une crème ou d'un gel moussant de nettoyage des cheveux.

12. Procédé de traitement non-thérapeutique des cheveux dans lequel on applique sur lesdites matières kératiniques une composition cosmétique telle que définie à l'une des revendications 1 à 11, et éventuellement à rincer lesdites matières kératiniques.

## Patentansprüche

1. Zusammensetzung, umfassend:
- (i) mindestens eine Verbindung der allgemeinen Formel (I) oder eines ihrer Salze: 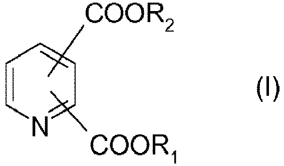 wobei R₁ und R₂ unabhängig voneinander für ein Wasserstoffatom, einen gesättigten oder ungesättigten, linearen oder verzweigten aliphatischen C₁-C₁₈-Kohlenwasserstoffrest (insbesondere Alkyl) oder einen C₆-C₁₈-Arylrest stehen;
wobei die Reste gegebenenfalls durch eine oder mehrere Gruppen, die aus OH, NH₂, -OR, -OCOR und -NHR ausgewählt sind, substituiert sind, wobei R für eine C₁-C₁₈-Alkylgruppe steht,
wobei mindestens eine der Gruppen R₁ und R₂ von einem Wasserstoffatom verschieden ist;
und
- (ii) mindestens ein fixierendes Polymer, das aus gegebenenfalls quaternisierten Vinylpyrrolidon/Dialkylaminoacrylat- oder -methacrylat-Copolymeren von einfach ungesättigten C₄-C₈-Carbonsäuren oder -Carbonsäureanhydriden und Acrylamidoalkylsulfonsäure-Homopolymeren ausgewählt ist.

2. Zusammensetzung nach Anspruch 1, wobei die Substituenten COOR₁ und COOR₂ respektive in der 2- und 3- oder 2- und 4-Position des Pyridinkerns, vorzugsweise in der 2- und 4-Position, stehen.

3. Zusammensetzung nach einem der vorhergehenden Ansprüche, wobei R₁ und R₂ gleich sind.

4. Zusammensetzung nach einem der vorhergehenden Ansprüche, wobei R₁ und R₂ unabhängig voneinander für ein Wasserstoffatom oder einen gesättigten, linearen oder verzweigten aliphatischen C₁-C₁₈-, besser C₁-C₁₀- oder sogar C₁-C₆-Kohlenwasserstoffrest, insbesondere -Alkylrest, der gegebenenfalls durch eine Alkoxy- oder Acyloxygruppe (OR oder OCOR, wobei R für einen C₁-C₁₈-Alkylrest steht) substituiert ist, stehen, wobei mindestens eine der Gruppen R₁ und R₂ von einem Wasserstoffatom verschieden ist.

5. Zusammensetzung nach einem der vorhergehenden Ansprüche, wobei R₁ und R₂ gleich sind und für einen gesättigten linearen C₁-C₁₈-, vorzugsweise C₁-C₁₀- und noch besser C₁-C₆-Alkylrest und ganz besonders einen Ethylrest stehen.

6. Zusammensetzung nach einem der vorhergehenden Ansprüche, die als Verbindung der Formel (I) eine der folgenden Verbindungen umfasst:
- Pyridin-2,4-dicarbonsäuredimethylester,
- Pyridin-2,3-dicarbonsäuredimethylester,
- Pyridin-2,4-dicarbonsäurediethylester,
- Pyridin-2,3-dicarbonsäurediethylester,
- Pyridin-2,5-dicarbonsäurediethylester,
- Pyridin-2,5-dicarbonsäuredimethylester,
- Pyridin-2,4-dicarbonsäurediisopropylester,
- Pyridin-2,4-dicarbonsäuredi(acetyloxymethyl)-ester (Derivat der Formel (I), das so beschaffen ist, dass R₁ und R₂ für -CH₂-O-COCH₃ stehen).

7. Zusammensetzung nach einem der vorhergehenden Ansprüche, wobei die Verbindung bzw. die Verbindungen der Formel (I) in einer Menge zwischen 0,001 und 10 Gew.-%, insbesondere von 0,01 bis 5 Gew.-%, bezogen auf das Gesamtgewicht der Zusammensetzung, vorliegt bzw. vorliegen.

8. Zusammensetzung nach einem der vorhergehenden Ansprüche, wobei das fixierende Polymer bzw. die fixierenden Polymere in einer Menge zwischen 0,01 und 10 Gew.-%, insbesondere 0,01 und 5 Gew.-%, noch besser zwischen 0,015 und 1 Gew.-%, bezogen auf das Gesamtgewicht der Zusammensetzung, vorliegt bzw. vorliegen.

9. Zusammensetzung nach einem der vorhergehenden Ansprüche, umfassend außerdem mindestens ein Vitamin B und/oder eines seiner Analoge oder Derivate, das insbesondere aus Vitamin B3, Vitamin B5, Vitamin B6 und Vitamin B8 oder einem ihrer Analoge oder Derivate und spezieller aus Calciumpanthotenat, Panthenol, Panthenylethylether, Panthenylethyletheracetat und Panthenoltriacetat sowie Mischungen davon ausgewählt ist.

10. Zusammensetzung nach Anspruch 9, umfassend das Vitamin B bzw. die Vitamine B, das Analog bzw. die Analogen und/oder das Derivat bzw. die Derivate in einer Menge zwischen 0,001 und 5 Gew.-%, insbesondere 0,005 und 2 Gew.-%, noch besser zwischen 0,01 und 1 Gew.-%, bezogen auf das Gesamtgewicht der Zusammensetzung.

11. Zusammensetzung nach einem der vorhergehenden Ansprüche in Form einer haarkosmetischen Zusammensetzung, wie einer Haarpflegelotion, eines Haarshampoos oder Haarconditioners, einer flüssigen oder festen Seife zur Reinigung der Kopfhaut, eines Produkts zur Gestaltung der Frisur, einer Behandlungsmaske, einer Creme oder eines schäumenden Gels zur Reinigung der Haare.

12. Verfahren zur nichttherapeutischen kosmetischen Behandlung von menschlichen Keratinmaterialien, bei dem man auf die Keratinmaterialien eine kosmetische Zusammensetzung gemäß einem der Ansprüche 1 bis 11 aufbringt und die Keratinmaterialien gegebenenfalls spült.

## Claims

1. Composition comprising:
- (i) at least one compound of general formula (I), or one of its salts: 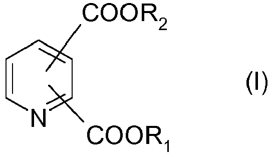 in which R₁ and R₂ represent, independently of one another, a hydrogen atom, a saturated or unsaturated and linear or branched aliphatic C₁-C₁₈ hydrocarbon radical (in particular alkyl) or a C₆-C₁₈ aryl radical;
said aliphatic or aryl hydrocarbon radicals optionally being substituted by one or more groups chosen from OH, NH₂, -OR, -OCOR and -NHR with R representing a C₁-C₁₈ alkyl group,
at least one of the R₁ and R₂ groups being other than a hydrogen atom;
and
- (ii) at least one fixing polymer chosen from quaternized or non-quaternized vinylpyrrolidone/dialkylaminoalkyl acrylate or methacrylate copolymers, copolymers of monounsaturated C₄-C₈ carboxylic acids or anhydrides or acrylamidoalkylsulfonic homopolymers.

2. Composition according to Claim 1, in which the COOR₁ and COOR₂ substituents are respectively in the 2 and 3, or 2 and 4, positions of the pyridine nucleus, preferably in the 2 and 4 positions.

3. Composition according to either of the preceding claims, in which R₁ and R₂ are identical.

4. Composition according to one of the preceding claims, in which R₁ and R₂ represent, independently of one another, a hydrogen atom or a saturated, linear or branched, aliphatic C₁-C₁₈, better still C₁-C₁₀, indeed even C₁-C₆, hydrocarbon radical, in particular alkyl radical, which is optionally substituted by an alkoxy or acyloxy group (OR or OCOR with R denoting a C₁-C₁₈ alkyl radical), at least one of the R₁ and R₂ groups being other than a hydrogen atom.

5. Composition according to one of the preceding claims, in which R₁ and R₂ are identical and represent a saturated linear C₁-C₁₈, preferably C₁-C₁₀ and better still C₁-C₆ alkyl radical and very particularly an ethyl radical.

6. Composition according to one of the preceding claims, comprising, as compound of formula (I), one of the following compounds:
- dimethyl pyridine-2,4-dicarboxylate,
- dimethyl pyridine-2,3-dicarboxylate,
- diethyl pyridine-2,4-dicarboxylate,
- diethyl pyridine-2,3-dicarboxylate,
- diethyl pyridine-2,5-dicarboxylate,
- dimethyl pyridine-2,5-dicarboxylate,
- diisopropyl pyridine-2,4-dicarboxylate,
- di(acetyloxymethyl) pyridine-2,4-dicarboxylate (derivative of formula (I) such that R₁ and R₂ represent -CH₂-O-COCH₃).

7. Composition according to one of the preceding claims, in which the compound or compounds of formula (I) are present in an amount of between 0.001% and 10% by weight, in particular from 0.01% to 5% by weight, with respect to the total weight of the composition.

8. Composition according to one of the preceding claims, in which the fixing polymer or polymers are present in an amount of between 0.01% and 10% by weight, in particular 0.01% and 5% by weight and better still between 0.015% and 1% by weight, with respect to the total weight of the composition.

9. Composition according to one of the preceding claims, additionally comprising at least one vitamin B and/or one of its analogues or derivatives chosen in particular from vitamin B3, vitamin B5, vitamin B6 and vitamin B8 or one of their analogues or derivatives and more particularly from calcium pantothenate, panthenol, panthenyl ethyl ether, panthenyl ethyl ether acetate and panthenyl triacetate, and also their mixtures.

10. Composition according to Claim 9, comprising the vitamin or vitamins B, analogue(s) and/or derivative(s) in an amount of between 0.001% and 5% by weight, in particular 0.005% and 2% by weight and better still between 0.01% and 1% by weight, with respect to the total weight of the composition.

11. Composition according to one of the preceding claims, which is provided in the form of a hair cosmetic composition, such as a hair care lotion, of a shampoo or of a hair conditioner, of a liquid or solid soap for cleaning the scalp, of a product for shaping the hairstyle, of a treatment mask, of a cream or of a foaming gel for cleaning the hair.

12. Method for the non-therapeutic treatment of the hair, in which a cosmetic composition as defined in one of Claims 1 to 11 is applied to said keratinous substances and said keratinous substances are optionally rinsed.
